# EUROPÄISCHE PATENTSCHRIFT

(11) **EP 1 492 591 B1**
(45) Veröffentlichungstag und Bekanntmachung des Hinweises auf die Patenterteilung: **03.12.2008**
(21) Anmeldenummer: 03722435.9
(22) Anmeldetag: 09.04.2003
(51) Int. Cl.: A61N 1/00

(54) **VORRICHTUNG ZUR FÖRDERUNG DES KNOCHENWACHSTUMS, INSBESONDERE ZUR OSTEOSYNTHESE VON KNOCHENFRAGMENTEN UND/ODER FIXATION VON KNOCHENFRAKTUREN**
DEVICE FOR STIMULATING BONE GROWTH, ESPECIALLY FOR THE OSTEOSYNTHESIS OF BONE FRAGMENTS AND/OR FOR FIXING BONE FRACTURES
DISPOSITIF POUR FAVORISER LA CROISSANCE OSSEUSE, NOTAMMENT POUR LA SYNTHESE OSSEUSE DE FRAGMENTS D'OS ET/OU LA FIXATION DE FRACTURES OSSEUSES

(30) Priorität: 11.04.2002 DE 10215996
(43) Veröffentlichungstag der Anmeldung: 05.01.2005
(73) Patentinhaber: Gundolf, Ferdinand, A-6330 Kufstein (AT)
(72) Erfinder: Gundolf, Ferdinand, A-6330 Kufstein (AT)
(74) Vertreter: Popp, Eugen
(86) Internationale Anmeldenummer: PCT/EP2003/003690
(87) Internationale Veröffentlichungsnummer: WO 2003/084602

(56) Entgegenhaltungen:
- EP-A- 1 023 872
- DE-A- 19 508 753
- US-A- 4 216 548

## Beschreibung

Die Erfindung betrifft eine Vorrichtung zur Förderung des Knochenwachstums, insbesondere zur Osteosynthese von Knochenfragmenten und/oder Fixation von Knochenfrakturen.

Im vorliegenden Fall geht es also um die Förderung von Knochenwachstum, insbesondere im Bereich von Knochenfrakturen, aber auch zur Reduzierung von Osteoporose, welche zunehmend eine volkswirtschaftliche Herausforderung darstellt. Allein in Österreich müssen zum Beispiel 150 Millionen Euro pro Jahr für die Versorgung von Schenkelhalsbrüchen aufgebracht werden, wobei die Folgekosten dabei noch nicht berücksichtigt sind. Im Schnitt erkrankt jede dritte Frau im Alter von 60 bis 70 Jahren an Osteoporose. Bei den über 80-jährigen sind sogar 2/3 aller Frauen betroffen. Osteoporose-bedingte Frakturen führen zu Immobilität und Pflegebedürftigkeit, zu Schmerzen und Verlust von Lebensqualität. Die Mortalität während der Rehabilitationsphase ist hoch. Die medizinischen Kosten für die Behandlung von Osteoporose-bedingten Frakturen, die derzeit jährlich in den USA und Europa aufgebracht werden müssen, betragen ca. 25 Milliarden Euro. In diesem Betrag sind die indirekten Folgekosten nicht enthalten, wie etwa die Kosten für Rehabilitation und Pflege, für Krankenstände, Arbeitsausfälle und institutionelle Dauerpflege.

Dementsprechend besteht ein enormes Bedürfnis nach Abhilfe und Reduzierung der vorgenannten Kosten.

Aus der DE 4 102 462 A1, die auf den Erfinder zurückgeht, ist bereits eine rein mechanische Vorrichtung zur Förderung des Knochenwachstums bekannt. Das dort beschriebene in der Regel längliche Stabilisationselement zur Osteosynthese von Knochenfragmenten weist trotz dünnwandiger Bauweise eine hohe Steifigkeit auf, und zwar bedingt durch einen Bogen-, Wellen-, mäander-, zick-zack- od. dgl. -förmigen Querschnitt. Die Praxis hat gezeigt, dass das erwähnte Stabilisationselement außerdem verträglich und zudem einfach zu implementieren ist. Als besonders geeignet erweist sich das erwähnte Stabilisationselement zur mechanischen Stützung bzw. Unterstützung bei der Heilung komplizierter Knochenbrüche. Dadurch, dass das bekannte Stabilisationselement nur Linienkontakt mit dem zugeordneten Knochen besitzt sowie aus biologisch verträglichem Material, wie beispielsweise Titan oder Titanlegierung hergestellt ist, welches an der Oberfläche vorzugsweise aufgeraut ist, wird das Knochenwachstum positiv gefördert.

Alternative Untersuchungen haben gezeigt, dass das Knochenwachstum weiterhin durch eine elektrische Anregung gefördert werden kann. Hierzu werden bisher zwei verschiedene Methoden eingesetzt:
Einerseits kann die elektrische Anregung direkt mittels galvanischer Kopplung über Zuführungsdrähte oder andererseits induktiv über ein äußeres elektromagnetisches Feld erfolgen.

Die direkte (galvanische) Anregung weist den Nachteil auf, dass eine Folge elektrischer Impulse transkutan über Zuführungsdrähte von außerhalb des Körpers durch die Haut eines Patienten hindurch erfolgt. Die Zuführungsdrähte müssen zudem nach Heilung des Knochens operativ wieder entfernt werden. Für die induktive Stimulation ist ein erheblicher Aufwand an externen Apparaten zur Erzeugung von elektromagnetischen Feldern erforderlich.

In der praktischen Anwendung weisen die beiden vorgenannten Verfahren zur elektrischen Stimulation des Knochenwachstums einen weiteren wesentlichen Nachteil auf: Aufgrund der externen Einrichtungen, wie eine elektrische Impulsquelle zur Erzeugung von elektrischen Anregungsimpulsen, Zuführungsdrähten und dergleichen Mittel können die beiden Verfahren nur unter Aufsicht beispielsweise einer Arztpraxis oder einem Krankenhaus durchgeführt werden. Dadurch ist die Anwendung auf bestimmte Zeiten beschränkt. Für eine besondere schnelle Heilung wäre jedoch eine Applikation der Verfahren ohne zeitliche Beschränkung und auf Sicht günstig.

Schließlich wird bei den beiden vorgenannten Verfahren der Bewegungszustand eines Patienten nicht berücksichtigt. Die Verfahren sind in keiner Weise adaptiv und wirken auf den Patienten ohne Rücksichtnahme auf dessen Absorptionsfähigkeit - medizinisch auch als "Resonanzfähigkeit" bezeichnet - ein. In diesem Zusammenhang wäre beispielsweise eine Adaption von Intensität und Frequenz elektrischer Impulse zur Stimulation des Knochenwachstum in Abhängigkeit von der Belastung des zu heilenden Knochen wünschenswert.

Die vorgenannten Nachteile lassen sich vermeiden durch eine Vorrichtung, die mindestens ein einem Implantat oder dem Knochen unmittelbar zugeordnetes piezo-elektrisches Element umfasst, sowie dies in der US 6 143 035 oder korrespondierenden EP 1 023 872 A2 beschrieben ist. Dieser Vorschlag vermeidet externe Apparate und/oder Zuführungsdrähte von einer externen elektrischen Impulsquelle. Außerdem hat der bekannte Vorschlag den Vorteil, dass die vom piezo-elektrischen Element erzeugten elektrischen Impulse zur Stimulation des Knochenwachstums adaptiv sind, d.h. die Stimulation richtet sich nach dem aktuellen Bewegungs- und Belastungszustand eines Patienten.

Nachteilig ist bei dem Vorschlag nach der US 6 143 035 die Anbringung des oder der piezo-elektrischen Elemente an der Außenseite eines Implantats, zum Beispiel Hüftschaftes. Das oder die piezo-elektrischen Elemente stehen dementsprechend über das Implantat nach außen vor. Damit verliert das Implantat die ursprüngliche Passgenauigkeit mit der Folge, dass die Gefahr einer Lockerung besteht. Außerdem besteht die Gefahr, dass das oder die piezo-elektrischen Elemente aufgrund äußerer Belastungen vom Implantat abgetrennt und damit unwirksam werden. Bei der Anbringung eines piezo-elektrischen Elements an der Außenfläche eines Implantats lässt sich auch die notwendige maximale Toleranz von 0,1 bis 0,25 mm über die gesamte Oberfläche des Implantats in Relation zu einem vorgeraspelten Implantat-Aufnahmeraum nicht mehr einhalten.

Dementsprechend liegt der vorliegenden Erfindung die Aufgabe zugrunde, eine Vorrichtung der eingangs genannten Art zu schaffen, bei der piezo-elektrische Elemente über die ImplantatOberfläche nicht vorstehen, so dass das Implantat in herkömmlicher Weise implantiert werden kann. Außerdem soll gewährleistet sein, dass die auf das Implantat einwirkenden Kräfte unmittelbar auf das dem Implantat zugeordnete piezo-elektrische Element einwirken.

Diese Aufgabe wird dadurch gelöst, dass das wenigstens eine piezo-elektrische Element integraler Bestandteil des Implantats ist.

Dadurch, dass das piezo-elektrische Element integraler Bestandteil des Implantats ist, soll sichergestellt sein, dass die äußere Kontur des Implantats unverändert bleibt. Dementsprechend lässt sich das Implantat in herkömmlicher Weise implantieren. Durch die Einbettung des piezo-elektrischen Elements innerhalb des Implantats ist auch eine dauerhaft unmittelbare Einwirkung von äußeren Kräften auf das piezo-elektrische Element über das Implantat gewährleistet. Das Implantat definiert stets den einen elektrischen Pol, insbesondere Minuspol, des piezo-elektrischen Elements, wobei der zweite Pol, insbesondere der Pluspol, durch ein nur mit dem umliegenden Knochen in Berührung kommendes Kontaktelement aus humanverträglichem, elektrisch leitendem, insbesondere metallischem Werkstoff definiert ist. Vorzugsweise besteht dieses Kontaktelement ebenfalls aus Implantatwerkstoff. Es kann streifen-, scheiben- oder knopfartig ausgebildet sein. Dies hängt letztlich von der Geometrie der Öffnung der Aufnahmetasche für das piezo-elektrische Element im Implantat ab.

Um sicherzustellen, dass das Implantat trotz des integrierten piezo-elektrischen Elements seine ursprüngliche Kontur beibehält, ist das piezo-elektrische Element innerhalb einer zum Knochen hin offenen Implantattasche angeordnet, insbesondere derart, dass es im wesentlichen bündig mit der Implantatoberfläche abschließt.

Die Erfindung ist in den Patentansprüchen definiert.

Spezielle Ausführungsformen von Implantaten mit zugeordneten piezo-elektrischen Elementen sind in den Ansprüchen 2 ff. beschrieben. Sie werden nachstehend auch noch näher anhand der beigefügten Zeichnung erläutert. Diese zeigen in
- Figur 1: ein Zahnimplantat mit piezo-elektrischem Element teilweise im Längsschnitt, teilweise in Seitenansicht;
- Figur 2: das Zahnimplantat gemäß Figur 1 im Quer- schnitt Längslinie III-III in Figur 1;
- Figur 3: einen knocheninduzierenden Schenkelhalsstift teilweise in Seitenansicht, teilweise im Längsschnitt unter Darstellung der Implanta- tion innerhalb eines Schenkelhalses;
- Figur 4: eine Hüftgelenkpfanne mit knocheninduzieren- dem piezo-elektrischen Element im Schnitt;
- Figur 5: einen Hüftschaft in Seitenansicht mit einem piezo-elektrischen Element an der anterio- ren/posterioren Seite einerseits und einem weiteren piezo-elektrischen Element lateral;
- Figur 6 und 7: den Hüftschaft gemäß Figur 5 im Querschnitt Längslinie VI-VI sowie Längslinie VII-VII in Figur 5;
- Figur 8: einen Femurschlitten im Schnitt unter Dar- stellung Befestigungsschrauben mit piezo- elektrischem Element;
- Figur 9: ein Tibiateil im Schnitt, dessen Knochen- Befestigungsschrauben jeweils mit einem pie- zo-elektrischen Element versehen sind; und
- Figur 10: einen Querschnitt durch ein halbkreisförmi- ges Stabelement zur Stabilisierung einer Knochenfraktur, dessen dem Knochen zugewand- ter Hohlraum mit einem piezo-elektrischen Element ausgefüllt ist, im Querschnitt.

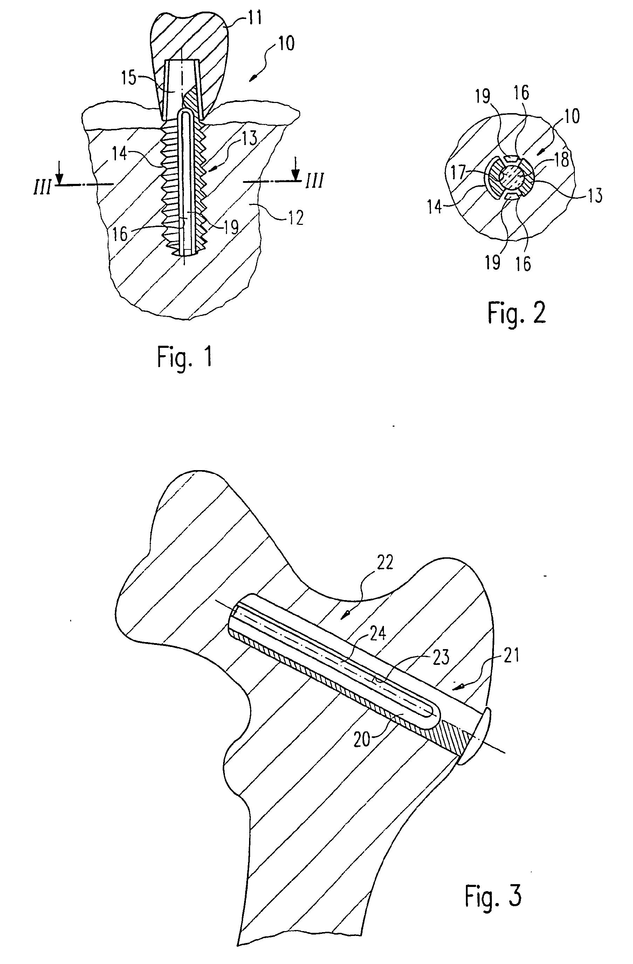

In den Figuren 1 und 2 ist im Teil Längs- und Querschnitt ein Zahnimplantat 10 für einen künstlichen Zahn 11 dargestellt. Der im (Kiefer-)Knochen 12 verankerbare Teil ist als Knochenschraube 13 mit Außengewinde 14 ausgebildet, wobei der obere Teil der Knochenschraube 13 einen Konus 15 umfaßt, auf den der künstliche Zahn 11 aufgesteckt werden kann. Der Gewindeteil der Knochenschraube 13 ist hohl ausgebildet und mit zwei diametral zueinander angeordneten Längsschlitzen 16 versehen. Der Längshohlraum 17 des Gewindeabschnitts der Knochenschraube 13 ist mit piezo-elektrischer Keramik, die das erfindungsgemäße piezo-elektrische Element definiert, ausgefüllt. Im Bereich der beiden Längsschlitze 16 erstrecken sich elektrisch leitende Kontaktstreifen 19, die vorzugsweise aus demselben Werkstoff hergestellt sind wie die Knochenschraube 13, nämlich aus Titan oder einer Titanlegierung, also einem Werkstoff, der humanverträglich ist. Die Kontaktstreifen 19 stehen nur mit dem Knochen 12 einerseits und der piezo-elektrischen Keramik 18 andererseits in Berührung, also nicht mit der Implantatschraube 13. Dementsprechend definieren die Kontaktstreifen 19 den Gegenpol zu der Knochenschraube 13. Letztere bildet vorzugsweise den Minuspol, während die Kontaktstreifen 19 den Pluspol definieren.

Durch die Kontaktstreifen 19 werden die Längsschlitze 16 im wesentlichen ausgefüllt, so dass die ursprüngliche Außenkontur der Knochenschraube 13 nahezu unverändert ist. Das beschriebene Zahnimplantat ist besonders hoch wirksam, und zwar aufgrund der dynamischen Belastung des piezo-elektrischen Elements 18 beim Kauen. Die dabei erzeugten elektrischen Signale bzw. Impulse bewirken eine schnellere Heilung des Kieferknochens 12.

Aus bisher gewonnenen Erkenntnissen mit der elektrischen Anregung der Knochenheilung ist es bekannt, dass eine effektive Stromstärke (Gleich-, Wechsel- oder Rechteckimpulsstrom) von etwa 10-100 µA für die Förderung des Knochenwachstums am besten ist. Vorzugsweise ist daher das piezo-elektrische Element derart ausgebildet, dass bei einer üblichen Belastung der Knochenstruktur ein Strom mit einer effektiven Stromstärke von etwa 10-100 µA erzeugt wird.

Das piezo-elektrische Element 18 besteht vorzugsweise aus einer piezo-elektrischen Keramik. Zirkunat oder Titanat-Keramiken haben sich hierbei als besonders geeignet für den chirurgischen/orthopädischen Anwendungsbereich erwiesen, da diese körperverträglich und einfach in den Körper integrierbar sind. Andere körperverträgliche und piezo-elektrisch aktive Keramiken, wie Quarzkeramik, sind ebenfalls denkbar.

In Figur 3 ist die Implantation eines Schenkelhalsstiftes 21 mit piezo-elektrischem Element 20 im Bereich eines frakturgefährdeten Schenkelhalses im Teil-Längsschnitt und Teil-Seitenansicht dargestellt. Der entsprechend gefährdete Schenkelhals ist mit der Bezugsziffer 22 gekennzeichnet. Der Schenkelhalsstift 21, der aus Titan oder einer Titanlegierung besteht, ist ähnlich wie die Knochenschraube 13 hohl ausgebildet. Der Hohlraum ist mit piezo-elektrischer Keramik ausgefüllt, die das piezo-elektrische Element 20 definiert. Auch hier sind zwei diametral zueinander angeordnete Längsschlitze 23 vorgesehen, in deren Bereich elektrisch leitende Kontaktstreifen 24 entsprechend den Kontaktstreifen 19 gemäß den Figuren 1 und 2 platziert sind, und zwar so, dass sie einerseits mit der Keramik 20 und andererseits mit dem umgebenden Knochen in Berührung stehen. Die Kontaktstreifen 24 bilden also dann den Gegenpol zu dem Stift 21. Es sei an dieser Stelle erwähnt, dass der Knochenaufbau eine kristalline Struktur aufweist und bei mechanischer Belastung mit piezo-elektrischen Impulsen reagiert. Im umgekehrten Fall reagiert der Knochen mit einem mechanischen Moment. Das wiederum führt zu Knochenaufbau. Dieses gegenseitige Einwirken von mechanischen Momenten und piezo-elektrischen Impulsen wird erfindungsgemäß ausgenutzt. Der in Figur 3 dargestellte Schenkelhalsstift dient hier zur Prophylaxe. Er kann jedoch genauso gut zur Heilung einer Schenkelhalsfraktur dienen.

In ganz ähnlicher Weise können zur Prophylaxe Knochen- bzw. Pedikelschrauben an anderen Stellen des Knochens eingebracht werden. Derartige Knochen- bzw. Pedikelschrauben weisen einen Gewindeteil auf entsprechend demjenigen der Knochenschraube 13 in den Figuren 1 und 2.

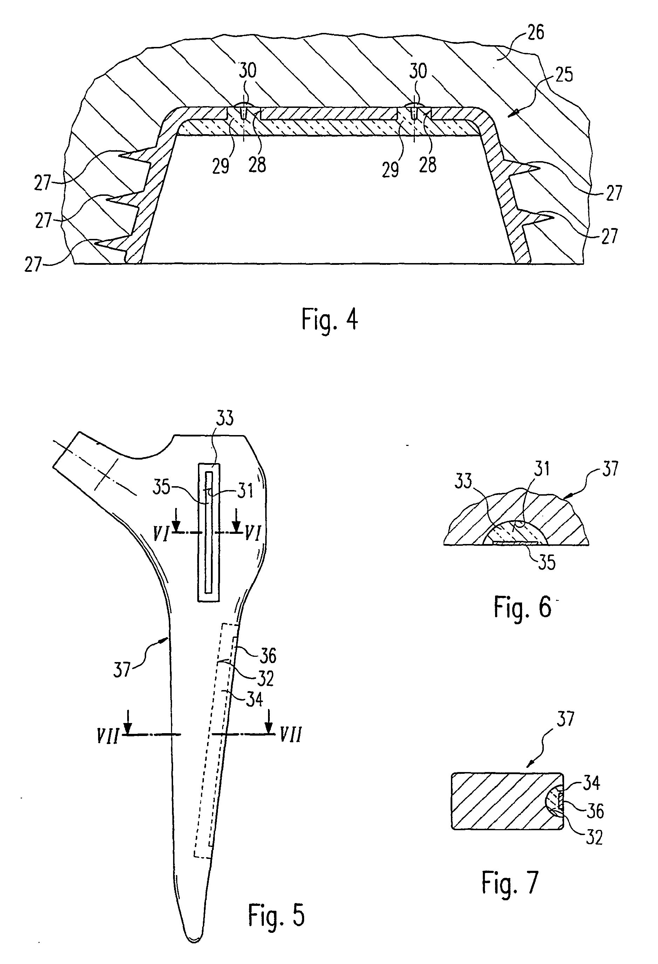

In Figur 4 ist eine Hüftgelenk- bzw. Hüftpfanne 25 dargestellt, die im Beckenknochen 26 eingeschraubt ist. Das entsprechende Schraubgewinde ist in Figur 4 mit der Bezugsziffer 27 gekennzeichnet. Bei alten und sehr alten Patienten retrahiert sich der Knochen im Bereich einer zementlos implantierten Hüftpfanne, wie die hier dargestellte Hüftpfanne 25. Die Hüftpfanne wird dann nur mehr von zarten Knochenbälkchen in Lage gehalten. Um diesem Problem vorzubeugen, sind bei der dargestellten Hüftpfanne 25 Bodenöffnungen 28 vorgesehen, die jeweils mit piezo-elektrischer Keramik 29 ausgefüllt sind. Die piezo-elektrische Keramik erstreckt sich dabei auch noch über die gesamte Innenseite des Pfannenbodens und wird durch das in Figur 4 nicht dargestellte Inlay druckbeaufschlagt. Pfannen-außenseitig steht die piezo-elektrische Keramik 29 über druckknopfartige Kontaktelemente 30 mit dem Knochen 26 in Berührung. Die druckknopfartigen Kontaktelemente 30 stehen mit der piezo-elektrischen Keramik 29 einerseits und dem umgebenden Knochen 26 andererseits in Kontakt; im übrigen sind sie gegenüber der Pfanne 25 isoliert. Die Kontaktelemente 30 bilden somit den elektrischen Gegenpol zur Pfanne 25. Das Knochenwachstum wird bei der dargestellten zementlos implantierten Hüftpfanne zum einen durch die Spitzen des Gewindes 27 und zum anderen durch das dargestellte piezo-elektrische System gefördert. Insbesondere durch letzteres erfolgt eine Knocheninduktion zum Implantat hin, welches im Laufe der Zeit zunehmend stabilisiert wird. Durch die beschriebenen Maßnahmen tritt also genau der umgekehrte Effekt ein, der üblicherweise zu erwarten wäre, nämlich Knochenaufbau statt Knochenabbau.

Die Figuren 5 bis 7 zeigen einen Hüftschaft mit anterior sowie lateral ausgebildeter Tasche 31 bzw. 32 zur Aufnahme einer piezo-elektrischen Keramik bzw. eines piezo-elektrischen Elements 33 bzw. 34. Die Aufnahmetaschen 31 bzw. 32 sind jeweils längsnutartig ausgebildet. Sie weisen jeweils einen etwa halbkreisförmigen Querschnitt auf. An der dem Knochen zugewandten Seite sind in die piezo-elektrische Keramik 33 bzw. 34 jeweils Kontaktstreifen 35 bzw. 36 eingebettet, und zwar so, dass die piezo-elektrische Keramik einschließlich Kontaktstreifen bündig mit der Außenoberfläche des Implantats, hier Hüftschaftes 37 abschließen.

Der Hüftschaft 37 kann auch an der posterioren und/oder medialen Seite noch mit piezo-elektrischen Elementen entsprechenden Elementen 33, 34 versehen sein. Letztlich hängt dies vom Knochengefüge des Patienten ab. Auch hier bilden die Kontaktstreifen 35, 36 wieder jeweils den elektrischen Pluspol des piezo-elektrischen Elements 33 bzw. 34, während das Implantat selbst, nämlich der Hüftschaft 37 den Minuspol definiert.

Die beschriebenen Beispiele zeigen auch sehr deutlich, dass keine Drähte zur Übertragung von Stromimpulsen verlegt sind. Die Implantate sollen im wesentlichen ihre ursprüngliche Form aufweisen, so dass sie in herkömmlicher Weise implantiert werden können.

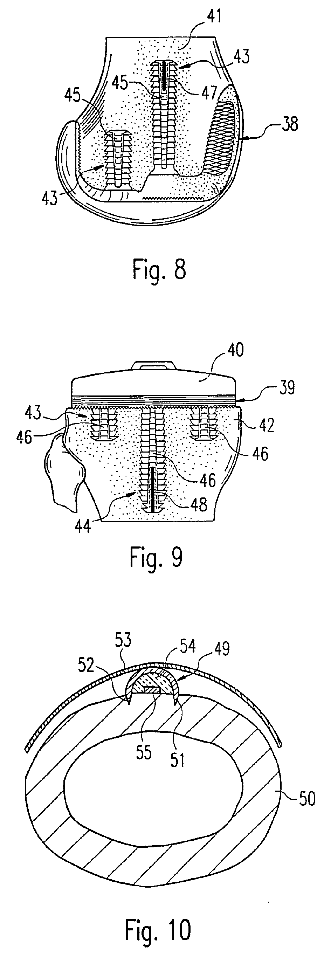

In den Figuren 8 und 9 ist im schematischen Längsschnitt ein Femurschlitten 38 einerseits und ein Tibiaplateau 39 andererseits dargestellt, wobei auf letzterem ein Lagerkörper 40 aus Polyethylen od. dgl. humanverträglichem Kunststoff fest oder verschieblich (Translation und/oder Rotation) gelagert ist.

Sowohl der Femurschlitten als auch das Tibiaplateau werden am Femur 41 bzw. an der Tibia 42 mittels Knochenschrauben 43 bzw. 44 befestigt. Die Knochenschrauben 43, 44 weisen einen Gewindeteil auf, welcher demjenigen der Knochenschraube 13 gemäß Figur 1 entspricht. Auch bei den Knochenschrauben 43, 44 ist ein Längshohlraum vorgesehen, der mit einer piezo-elektrischen Keramik ausgefüllt ist. Diese bildet jeweils ein piezo-elektrisches Element 45, 46. Im Bereich der Längsschlitze sind wiederum Kontaktstreifen 47, 48 vorgesehen, die mit der piezo-elektrischen Keramik einerseits und dem umgebenden Knochen andererseits in Berührung stehen.

Das piezo-elektrische Element 45 bzw. 46 ist jeweils etwas konisch ausgebildet, und zwar in Richtung zum Schraubenende hin konisch erweitert, so dass der Gewindeteil der Knochenschrauben 43, 44 entsprechend radial nach außen gespreizt wird. Dadurch wird ein besserer Halt in der Knochen-Spongiosa erreicht.

In Figur 10 ist im Querschnitt ein längliches Stabilisationselement gemäß der DE 4 102 462 A1 dargestellt, und zwar in Zuordnung zu einem Knochen 50. Das Stabilisationselement, welches als längliches Halbrohr ausgeführt ist, ist mit der Bezugsziffer 49 gekennzeichnet. Es hat mit der Knochenoberfläche nur Linienkontakt, wobei dieser Linienkontakt durch im Längsabstand voneinander angeordnete Spitzen 51, 52 unterbrochen ist, die in den Knochen eindringen. Das dargestellte Stabilisationselement wird durch ein um den Knochen und das Stabilisationselement 49 herumgeschlungenes Halteband 53 in Position gehalten. Das Halteband 53 ist in Figur 10 nur zum Teil dargestellt. Vor allem fehlt die Darstellung des Schließelements für die beiden freien Enden des Haltebandes. Diesbezüglich handelt es sich jedoch um bekannten Stand der Technik, der ebenfalls auf den Erfinder zurückgeht.

Der Hohlraum zwischen Stabilisationselement 49 und Knochenoberfläche ist mit einer piezo-elektrischen Keramik ausgefüllt. An der der Knochenoberfläche zugewandten Seite ist in die Keramik 54 ein elektrisch leitender Kontaktstreifen 55 eingebettet. Dieser ist wie bei den vorbeschriebenen Ausführungsformen gegenüber dem Stabilisationselement durch die Keramik 54 isoliert und definiert den Gegenpol zum Stabilisationselement 49, welches aus Titan oder einer Titanlegierung besteht. Auch hier ist also das piezo-elektrische Element 54 integraler Teil des Stabilisationselements 49.

Bei Zuordnung von mehr als einem piezo-elektrischen Element können mindestens zwei piezo-elektrische Elemente zur Erzielung einer höheren elektrischen Spannung elektrisch in Reihe geschaltet werden. Alternativ können die piezo-elektrischen Elemente auch elektrisch parallel geschaltet werden, wodurch eine höhere Stromstärke erzielbar ist. Wesentlich ist, dass die effektive Stromstärke von 10-100 µA erreicht wird. In entsprechender Weise sind die piezo-elektrischen Elemente dann zu schalten.

Wie bereits erwähnt, können die piezo-elektrischen Elemente den verschiedensten Implantaten zugeordnet werden, zum Beispiel auch einer künstlichen Patella. Insofern bestehen keine Grenzen.

Die Beaufschlagung des piezo-elektrischen Elements erfolgt jeweils über das Implantat einerseits und den Knochen andererseits, wobei auch Druck durch die Muskulatur ausgeübt werden kann.

### Bezugszeichenliste

- 10: Zahnimplantat
- 11: künstlicher Zahn
- 12: (Kiefer-) Knochen
- 13: Knochenschraube
- 14: Außengewinde
- 15: Steckkonus
- 16: Längsschlitz
- 17: Längshohlraum
- 18: piezo-elektrisches Element (Keramik)
- 19: Kontaktstreifen
- 20: piezo-elektrisches Element (Keramik)
- 21: Schenkelhalsstift
- 22: Schenkelhals
- 23: Längsschlitz
- 24: Kontaktstreifen
- 25: Hüftpfanne
- 26: Beckenknochen
- 27: Schraubgewinde
- 28: Bodenöffnung
- 29: piezo-elektrisches Element (Keramik)
- 30: Kontaktelement
- 31: Tasche
- 32: Tasche
- 33: piezo-elektrisches Element (Keramik)
- 34: piezo-elektrisches Element (Keramik)
- 35: Kontaktstreifen
- 36: Kontaktstreifen
- 37: Hüftschaft
- 38: Femurschlitten
- 39: Tibiaplateau
- 40: Lagerkörper
- 41: Femur
- 42: Tibia
- 43: Knochenschraube
- 44: Knochenschraube
- 45: piezo-elektrisches Element (Keramik)
- 46: piezo-elektrisches Element (Keramik)
- 47: Kontaktstreifen
- 48: Kontaktstreifen
- 49: Stabilisationselement
- 50: Knochen
- 51: Spitze
- 52: Spitze
- 53: Halteband
- 54: piezo-elektrisches Element (Keramik)
- 55: Kontaktstreifen

## Patentansprüche

1. Vorrichtung zur Förderung von Knochenwachstum, insbesondere zur Osteosynthese von Knochenfragmenten und/oder Fixation von Knochenfrakturen, welche zumindest ein Implantat (10; 21; 25; 37; 43; 44; 49), ein dem Implantat zugeordnetes piezo-elektrisches Element (18; 20; 29; 33; 34; 45; 46; 54), das unter der Einwirkung von Kräften elektrische Impulse erzeugt, die als Stimulans für das Knochenwachstum dienen, und ein nur mit umliegenden Knochen und dem piezo-elektrischen Element in Berührung kommendes Kontaktelement (19; 24; 30; 35; 36; 47; 48; 55) aus humanverträglichem, elektrisch leitendem, insbesondere metallischen Werkstoff umfasst,
wobei das wenigstens eine piezo-elektrische Element integraler Bestandteil des Implantats ist, das Implantat den einen Pol, insbesondere Minuspol, und
das Kontaktelement den anderen Pol, insbesondere Pluspol des piezo-elektrischen Elements definiert, und das piezo-elektrische Element (33; 34) innerhalb einer zum Knochen hin offenen Implantattasche (31; 32) angeordnet ist, insbesondere derart, daß es im wesentlichen bündig mit der Implantatoberfläche abschließt.

2. Vorrichtung nach Anspruch 1,
**dadurch gekennzeichnet, daß**
das Implantat (10; 21; 43; 44) nach Art eines Dübels ausgebildet ist, in dessen zentralem Hohlraum (17) das piezo-elektrische Element (18; 20; 45; 46) platziert ist.

3. Vorrichtung nach Anspruch 2,
**dadurch gekennzeichnet, daß**
das Implantat eine stiftartige Halterung für einen künstlichen Zahn (11), eine Knochen- oder Pedikelschraube (13; 43; 44), ein Knochenfixierstift (21) oder ein Knochenfixierelement (49) ist.

4. Vorrichtung nach Anspruch 1,
**dadurch gekennzeichnet, daß**
das Implantat eine Hüftgelenkpfanne (25) mit wenigstens einer Bodenöffnung (28) ist, wobei innerhalb dieser das piezo-elektrische Element (23) platzierbar ist.

5. Vorrichtung nach Anspruch 4,
**dadurch gekennzeichnet, daß**
das innerhalb der Bodenöffnung (28) angeordnete und diese ausfüllende piezo-elektrische Element (29) mit einer sich über zumindest einen Teil der Innenseite des Pfannenbodens erstreckenden piezo-elektrischen Schicht (29) integral verbunden ist.

6. Vorrichtung nach einem der Ansprüche 1 bis 5,
**dadurch gekennzeichnet, daß**
das piezo-elektrische Element derart ausgebildet ist, daß bei üblicher Belastung der Knochenstruktur ein Strom mit einer effektiven Stromstärke von etwa 10-100 µA erzeugbar ist.

7. Vorrichtung nach einem der Ansprüche 1 bis 6,
**dadurch gekennzeichet**, **daß**
das piezo-elektrische Element aus einer piezo-elektrischen Keramik, insbesondere einer Zirkonat- oder Titanat-Keramik hergestellt ist.

8. Vorrichtung nach einem der Ansprüche 1 bis 7,
**dadurch gekennzeichnet, daß**
bei mindestens zwei piezo-elektrischen Elementen diese entweder elektrisch in Reihe oder elektrisch parallel geschaltet sind.

## Claims

1. Device for promoting bone growth, in particular, the osteosynthesis of bone fragments and/or the fixation of bone fractures, which comprises:
at least one implant (10; 21; 25; 37; 43; 44; 49), a piezoelectric element (18; 20; 29; 33; 34; 45; 46; 54) associated with the implant, which, subject to the influence of forces, generates electrical pulses, which act as a stimulant for bone growth, and a contact element (19; 24; 30; 35; 36; 47; 48; 55) made of a human-compatible, electrically-conductive, especially metallic, material, which comes into contact exclusively with the surrounding bone and the piezoelectric element,
wherein the at least one piezoelectric element is an integral component of the implant,
the implant defines one pole, in particular, the minus pole, and the contact element defines the other pole, in particular, the plus pole of the piezoelectric element,
and the piezoelectric element (33; 34) is disposed within an implant pocket (31; 32) open towards the bone, in particular, in such a manner that it terminates substantially flush with the surface of the implant.

2. Device according to claim 1,
**characterised in that**
the implant (10; 21; 43; 44) is formed in the manner of a dowel pin, in the central hollow cavity (17) of which the piezoelectric element (18; 20; 45; 46) is positioned.

3. Device according to claim 2,
**characterised in that**
the implant is a pin-like holder for an artificial tooth (11), a bone or pedicle screw (13; 43; 44), a bone fixation pin (21) or a bone fixation element (49).

4. Device according to claim 1,
**characterised in that**
the implant is a hip-joint acetabulum (25) with at least one base opening (28), in which the piezoelectric element (29) can be placed.

5. Device according to claim 4,
**characterised in that**
the piezoelectric element (29), which is arranged within and fills the base opening (28), is connected in an integral manner to a piezoelectric layer (29) extending over at least one part of the internal side of the floor of the acetabulum.

6. Device according to any one of claims 1 to 5,
**characterised in that**
the piezoelectric element is formed in such a manner that, under normal loading of the bone structure, a current can be generated with an effective electric current of approximately 10-100 µA.

7. Device according to any one of claims 1 to 6,
**characterised in that**
the piezoelectric element is manufactured from a piezoelectric ceramic, in particular, a zirconate or titanate ceramic.

8. Device according to any one of claims 1 to 7,
**characterised in that,**
with at least two piezoelectric elements, these are connected either electrically in series or electrically in parallel.

## Revendications

1. Dispositif pour favoriser la croissance osseuse, en particulier l'ostéosynthèse de fragments d'os et/ou la fixation de fractures osseuses, qui comprend au moins un implant (10; 21; 25; 37; 43; 44; 49) et un élément piézoélectrique (18; 20; 29; 33; 34; 45; 46; 54) associé à l'implant qui, sous l'effet de forces, génère des impulsions électriques servant de stimulant de la croissance osseuse et un élément de contact (19; 24; 30; 35; 36; 47; 48; 55), constitué d'un matériau électriquement conducteur, en particulier métallique, compatible avec l'organisme humain, qui vient en contact uniquement avec l'os avoisinant et avec l'élément piézoélectrique, ledit ou lesdits éléments piézoélectriques faisant partie intégrante de l'implant, l'implant définissant un des pôles, en particulier le pôle négatif, et l'élément de contact l'autre pôle, en particulier le pôle positif, de l'élément piézoélectrique et l'élément piézoélectrique (33; 34) étant disposé à l'intérieur d'une poche d'implant (31; 32) ouverte en direction de l'os, en particulier de façon à être sensiblement affleurant par rapport à la surface de l'implant.

2. Dispositif selon la revendication 1, **caractérisé en ce que** l'implant (10; 21; 43; 44) est réalisé sous la forme d'une cheville dans le creux central (17) de laquelle l'élément piézoélectrique (18; 20; 45; 46) est placé.

3. Dispositif selon la revendication 2, **caractérisé en ce que** l'implant est un support en forme de pivot pour une dent artificielle (11), une vis à os ou une vis pédiculaire (13; 43; 44), une broche de fixation osseuse (21) ou un élément de fixation osseuse (49).

4. Dispositif selon la revendication 1, **caractérisé en ce que** l'implant est une cavité cotyloïde (25) dont le fond présente au moins une ouverture (28) à l'intérieur de laquelle l'élément piézoélectrique (29) peut être placé.

5. Dispositif selon la revendication 4, **caractérisé en ce que** l'élément piézoélectrique (29) disposé à l'intérieur de l'ouverture (28) du fond et la remplissant est relié d'un seul tenant à une couche piézoélectrique (29) qui s'étend sur une partie au moins de la face interne du fond de la cavité cotyloïde.

6. Dispositif selon l'une des revendications 1 à 5, **caractérisé en ce que** l'élément piézoélectrique est configuré de façon à générer un courant ayant une intensité efficace de 10 à 100 µA environ lors d'une sollicitation de la structure osseuse.

7. Dispositif selon l'une des revendications 1 à 6, **caractérisé en ce que** l'élément piézoélectrique est fabriqué en céramique piézoélectrique, en particulier une céramique à base de zirconate ou de titanate.

8. Dispositif selon l'une des revendications 1 à 7, **caractérisé en ce que,** lorsqu'il y a au moins deux éléments piézoélectriques, ceux-ci sont reliés électriquement soit en série, soit en parallèle.
